(19) Europäisches Patentamt
European Patent Office
Office européen des brevets

(11) EP 3 355 858 B1

(12) **FASCICULE DE BREVET EUROPEEN**

(45) Date de publication et mention de la délivrance du brevet:
**18.03.2020 Bulletin 2020/12**

(21) Numéro de dépôt: **16775631.1**

(22) Date de dépôt: **27.09.2016**

(51) Int Cl.:
***A61K 9/12*** *(2006.01)* ***A61K 31/498*** *(2006.01)*
***A61P 17/00*** *(2006.01)*

(86) Numéro de dépôt international:
**PCT/EP2016/073010**

(87) Numéro de publication internationale:
**WO 2017/055293 (06.04.2017 Gazette 2017/14)**

(54) **MOUSSE CHIMIQUE NON RINCÉE CONTENANT DE LA BRIMONIDINE ET SON UTILISATION DANS LE TRAITEMENT DE LA ROSACÉE**

SPÜLFREIER CHEMISCHER SCHAUM MIT BRIMONIDIN UND VERWENDUNG DAVON BEI DER BEHANDLUNG VON ROSACEA

NO-RINSE CHEMICAL FOAM CONTAINING BRIMONIDINE, AND USE THEREOF IN THE TREATMENT OF ROSACEA

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.09.2015 FR 1559200**

(43) Date de publication de la demande:
**08.08.2018 Bulletin 2018/32**

(73) Titulaire: **Galderma Research & Development**
**06410 Biot (FR)**

(72) Inventeurs:
- **BUGE, Jean-Christophe**
**06200 Nice (FR)**
- **NADAU-FOURCADE, Karine**
**06270 Villeneuve Loubet (FR)**

(74) Mandataire: **Casalonga**
**Casalonga & Partners**
**Bayerstraße 71/73**
**80335 München (DE)**

(56) Documents cités:
**WO-A1-00/27356 WO-A2-2012/001065**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 355 858 B1

## Description

La présente invention a pour objet un produit topique non rincé sous forme d'une mousse pour le traitement pharmaceutique de la peau, comprenant de la brimonidine sous forme solubilisée. La brimonidine est un agoniste des récepteurs alpha-2 adrénergiques indiqué dans le traitement par voie topique des érythèmes faciaux persistants dû à la rosacée.

Le terme "brimonidine" fait référence à la molécule (5-bromo-quinoxalin-6-yl)-(4,5-dihydro-1H-imidazol-2-yl)-amine ayant la structure suivante:

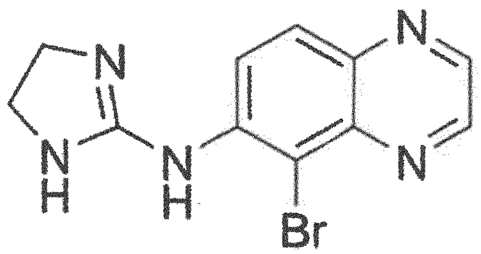

Malgré tous les progrès obtenus, le consommateur est toujours à la recherche de compositions destinées à une application topique plus pratique et rapide. Les mousses permettent de pallier les problèmes de tolérance par un meilleur contrôle de la dose, grâce à leurs propriétés d'étalement et leur faible densité.

La rosacée est une maladie inflammatoire chronique de la peau du visage parfois affichante qui peut avoir un impact important sur la vie des personnes touchées.

Elle se manifeste par des rougeurs sur le visage, principalement sur le front, les joues et le nez, accompagnées de boutons inflammatoires (papules et pustules). La peau atteinte de rosacée tend à être très sèche et sensible.

La peau qui souffre de rosacée est une peau très sensible et très réactive. Il faut éviter de frotter la peau, de la laver sous l'eau chaude de la douche. Des nettoyants doux sans rinçage, sans alcool et sans savon doivent être utilisés ainsi qu'une crème hydratante adaptée.

En outre, certains composés utilisés dans les compositions destinées à une application topique connue peuvent entraîner des effets secondaires qui peuvent en limiter l'utilisation et donc l'efficacité. Par exemple, certains actifs présentent l'inconvénient majeur d'induire de l'irritation pouvant entrainer une tolérance médiocre du produit. Cela peut ainsi créer de la part du patient un comportement de non observance du traitement et du mécontentement à l'égard dudit traitement.

Il existe donc un besoin de mettre au point de nouvelles formes galéniques palliant les inconvénients cités précédemment en termes de tolérance, d'efficacité et d'observance.

Dans ce genre de pathologie à long termes et pour lesquels on traite le symptômes sans savoir éradiquer la maladie, il existe donc le besoin de disposer de nouvelles formes galéniques et en particulier de type mousses ou compositions moussantes permettant un meilleur contrôle de la dose dans lesquelles la brimonidine est soluble, stable, bien tolérée, efficace et agréable à appliquer.

La composition selon l'invention présente l'avantage d'être sous forme d'une mousse générée de façon extemporanée, et très bien tolérée.

Après son application, la composition selon l'invention n'est pas éliminée par rinçage.

Un des avantages de la composition est d'être particulièrement bien tolérée, malgré le fait qu'elle ne soit pas éliminée par rinçage, comme le montrent les exemples illustrant une des méthodes d'évaluation de la tolérance présentés ci-après.

Il existe différentes méthodes d'évaluation de la tolérance d'un produit pharmaceutique ou cosmétique à usage cutané parmi lesquels on peut citer le test in vivo « in used » or « human patch test » mais aussi le test in vitro tel que le test de mesure de l'irritation sur Epiderme Humain Reconstruit (Reconstructed Human Epidermis ou RHE) décrit dans le protocole TG439 OCDE. Cette dernière méthode est détaillée dans l'exemple 3.

De plus, La composition présente l'avantage de rester en surface de la peau et délivrer une quantité moindre afin d'éviter les effets indésirables (effet rebond) et d'obtenir une mousse adaptée au traitement de la rosacée

L'efficacité d'un principe actif ses effets indésirables est lié à la pénétration du l'actif au travers de la peau. La composition formulaire joue son rôle primordial de véhicule du principe afin que celui-ci atteigne sa cible thérapeutique. Le test de libération - perméation décrit dans l'exemple 5 sur peau humaine ex-vivo met en avant l'avantage de l'application d'une mousse chimique contenant du tartrate de brimonidine.

Il existe actuellement des mousses ou compositions moussantes sur le marché. Toutefois, elles présentent toutes un certain nombre d'inconvénients :

En effet, il existe 3 types de mousses ou compositions moussantes :

- Des aérosols dans lesquels la mousse est générée par un gaz propulseur mais avec l'inconvénient d'être des aérosols présentant les risques bien connus de ceux-ci (pollution, risques respiratoires notamment).
- Des crèmes foisonnées dans lesquelles des bulles d'air sont introduites dans le produit par l'intermédiaire d'un procédé de fabrication particulier. Ce procédé présente l'inconvénient d'être contraignant au niveau industriel et nécessite un lourd investissement au niveau du matériel de conditionnement.
- Des formules moussantes peu riches en tensioactifs moussants mais conditionnées dans un emballage muni d'un système mécanique générateur de mousse (pompe avec grille de type pulvorex). Ce type de formulation nécessite l'utilisation de tensioactifs moussants pouvant induire de l'irritation dans des produits non rincés.

Ainsi, il subsiste donc le besoin de mettre au point une composition pharmaceutique dont la forme galénique est différente des formes galéniques connues afin, entre autres, de disposer de compositions destinées à une application topique contenant la brimonidine sous forme solubilisée dans des compositions bien tolérées destinées à une application topique chez l'être humain, en particulier une application non rincée (c'est-à-dire que la composition n'est pas éliminée par rinçage après son application).

Le but de la présente invention est donc de proposer une composition répondant à ces besoins.

La demanderesse a ainsi mis au point une nouvelle composition pharmaceutique, destinée à une application topique non rincée qui se présente sous la forme d'une mousse qui ne contient avantageusement pas de tensioactifs moussants. On définit par tensioactif moussant des tensioactifs qui produisent une mousse volumineuse, stable et onctueuse lorsqu'ils sont mélangés à l'eau selon les tests bien connus de l'homme du métier.

Constituent des tensioactifs moussants: les tensioactifs anioniques, les tensioactifs cationiques, les tensioactifs amphotères et les tensioactifs non ioniques de la famille des alkylpolyglucosides et des glucamides.

La forme galénique selon l'invention présente l'avantage de garantir une bonne solubilité et une bonne stabilité de la brimonidine. De plus, cette formulation conduit avantageusement à l'obtention d'une mousse douce, parfaitement tolérée et non irritante, qui permet une meilleure couverture de la zone à traiter et permet de pallier les problèmes de tolérance par un meilleur contrôle de la dose, grâce aux propriétés d'étalement et la faible densité de la mousse.

Enfin, de manière avantageuse, cette forme galénique ne nécessite pas pour sa mise en œuvre l'utilisation de gaz propulseurs ou d'aérosols. Ainsi les mousses dites aérosol ou spray sont exclues du champ de l'invention. De même, les mousses de l'art antérieur de type crèmes foisonnées et/ou formules moussantes nécessitant un système mécanique générateur de mousse (type pulvorex) sont également exclues de l'invention.

La présente invention a enfin pour objet un médicament destiné à une application topique sur la peau, comprenant une telle composition.

La présente invention sera décrite plus en détail dans la description et les exemples ci-après, et au vu des figures annexées à la présente demande.

La Figure 1 montre des photographies d'une première composition conforme à l'invention obtenue par mélange des deux compositions intermédiaires A5 placebo et B7 décrites dans les exemples, immédiatement après mélange de celles-ci puis lorsque la réaction entre ces deux compositions est complète (volume de mousse maximal).

Les figures 2A et 3A illustrent l'exemple 5 et représentent les résultats comparatifs de pénétration -perméation dans le stratum corneum, l'épiderme et le derme des compositions sous forme de mousse selon l'invention :

- Une composition commerciale de référence contenant 0.33% en poids de brimonidine sous forme de base correspondant à 0.5% en poids de tartrate de brimonidine ;
- Une Référence sous forme de gel contenant 0.12% en poids de brimonidine sous forme de base correspondant à 0.18% en poids de tartrate de brimonidine ;
- Une formule de mousse complète selon l'invention à 0.5% en poids de tartrate de brimonidine composée du mélange des compositions A3 et B 7 décrites dans l'exemple 1 (dans un ratio 50/50 en poids),
- Une formule de mousse complète selon l'invention à 0.18% en poids en poids de tartrate de brimonidine composée du mélange des compositions A 4 et B 7 décrites dans l'exemple 1 (dans un ratio 50/50 en poids).

Les figures 2B et 3B illustrent l'exemple 5 et représentent les résultats comparatifs de pénétration -perméation dans le derme à une échelle agrandie des figures 2A et 3A .

La composition selon l'invention est capable de prendre la forme d'une mousse par le seul fait de sa composition, et peut être également définie comme une composition auto-moussante pour application topique.

La présente invention a, en conséquence, pour premier objet une composition contenant de la brimonidine destinée à une application topique non rincée se présentant sous la forme d'une mousse, avantageusement de consistance semi-solide, ne contenant avantageusement pas de tensioactif moussant, et comprenant un milieu pharmaceutiquement compatible avec une application topique non rincée notamment sur la peau et les phanères.
Par composition se présentant sous la forme d'une mousse, (également dénommé ci-après composition auto-moussante), il est entendu une composition de consistance semi-solide ayant une forme aérée assimilable à une mousse.

La composition auto-moussante selon la présente invention comprend au moins deux compositions ou formules intermédiaires :

- au moins une composition intermédiaire B comprenant un agent générateur de gaz et au moins un agent gélifiant et/ou agent de suspensions choisi parmi les polymères d'acide acrylique, les polysaccharides, la famille des aluminium magnésium silicates, la famille des amidons modifiés et la famille des carraghénanes,
- au moins une composition intermédiaire A comprenant un agent activateur de l'agent générateur de gaz et au moins un agent gélifiant et/ou agent de suspensions choisi parmi les polysaccharides, la famille des aluminium magnésium silicates, la famille des amidons modifiés, la famille des carraghénanes et le PVA et
- de la brimonidine ou l'un de ses sels pharmaceutiquement acceptables contenue dans l'une au moins desdites formules intermédiaires A et B.

Selon l'invention, la composition est auto-moussante, c'est-à-dire qu'elle mousse par simple mélange des compositions intermédiaires A et B. L'invention a également pour objet la composition sous forme de mousse résultant du mélange desdites compositions intermédiaires A et B.

Selon l'invention, chaque composition (ou formule) intermédiaire peut présenter une viscosité (mesurée à 25°C et à pression atmosphérique) comprise entre 1 cP et 500000 cP, avantageusement entre 500 cP et 350000 cP, mesurée avec une méthode classique de type brookfield RV dv II : Aiguille 6 vit 2.

Selon l'invention le gaz généré par l'agent générateur de gaz peut être tout gaz physiologiquement compatible et permettant l'obtention d'une mousse, comme par exemple le dioxyde de carbone ($CO_2$) ou l'oxygène ($O_2$). De préférence, le gaz généré à partir de l'agent générateur de gaz est du dioxyde de carbone ($CO_2$).

Selon l'invention, la concentration en gaz pouvant varier, la quantité de bulles dans la composition peut varier et ainsi donner une composition pouvant aller de peu aérée à très fortement aérée.
Selon l'invention, on entend par agent activateur de l'agent générateur de gaz un ingrédient qui, par réaction chimique avec l'agent générateur de gaz, libère un gaz. Préférentiellement il s'agit d'une réaction acide/base.

Ainsi selon l'invention, la composition auto-moussante peut se présenter préférentiellement sous toutes les formes allant d'aérée à une mousse très expansée.

La composition selon l'invention est adaptée à une application topique et peut comprendre en outre un milieu physiologiquement acceptable, c'est-à-dire un milieu compatible avec la peau et les phanères. Il s'agit de préférence d'un milieu pharmaceutiquement acceptable.

En outre, la composition peut comprendre tout agent actif susceptible de présenter une activité, éventuellement thérapeutique. Ces agents actifs peuvent être, entre autres, choisis parmi les agents émollients, les agents humectants, les agents anti-radicalaires, les agents anti-inflammatoires, les vitamines, les agents dépigmentants, les agents anti-acnéiques, les agents anti-séborrhiques, les agents anti-fongiques, les agents kératolytiques, les filtres solaires, les agents amincissants, les agents de coloration de la peau.

Selon l'invention, la composition sous forme de mousse (c'est-à-dire prête à être appliquée) peut avoir un pH compris entre 2 et 8, préférentiellement entre 4 et 7.

Dans la mesure où la ou les composition(s) (ou formule(s)) intermédiaire(s) nécessite(nt) un stockage en au moins 2 compartiments pour des raisons de stabilité des ingrédients, la présente invention se rapporte soit à un unique contenant compartimenté (chaque compartiment accueillant une formule intermédiaire) et de préférence comprenant 2 ou 3 compartiments, soit à un kit comprenant chaque formule intermédiaire stockée indépendamment l'une de l'autre et physiquement séparées.
Le mélange intime extemporané (directement sur la peau ou sur tout autre support) des formules intermédiaires permet d'obtenir la composition sous forme de mousse selon l'invention.

De manière plus spécifique, la composition (ou formule) intermédiaire A peut se présenter sous forme d'une solution, d'une émulsion (lotion, crème, crème sans émulsionnant, lait, crème fluide) ou d'un gel. Cette composition contient avantageusement l'agent activateur de l'agent générateur de gaz, préférentiellement un acide en quantité suffisante (pouvant se présenter sous forme d'un tampon acide/Base à pH acide) qui peut être à titre d'exemple non limitatif le couple acide citrique/citrate de sodium.

La formule B peut se présenter sous forme d'une solution, d'un gel, d'une émulsion (lotion, crème, crème sans émulsionnant, lait, crème fluide). Cette composition contient avantageusement en quantité suffisante un agent générateur de gaz, qui peut être en particulier du bicarbonate de sodium.

Ainsi, l'invention a également pour objet un kit ou un contenant unique à plusieurs compartiments tel que défini précédemment, permettant la préparation extemporanée d'une composition sous forme de mousse selon l'invention, comprenant de manière séparée au moins deux formules intermédiaires (ou compositions intermédiaires):

- une formule intermédiaire A telle que définie ci-avant comprenant au moins un agent activateur de l'agent générateur de gaz ; et
- une formule intermédiaire B telle que définie ci-avant comprenant au moins un agent générateur de gaz ;
- de la brimonidine ou l'un de ses sels pharmaceutiquement acceptables étant contenue dans l'une au moins desdites formules intermédiaires A et B.

De préférence, la brimonidine est contenue dans la composition intermédiaire A.

AGENT ACTIVATEUR DE GAZ :

L'agent activateur de l'agent générateur de gaz (également désigné par « agent activateur de gaz ») est un composé qui réagit avec l'agent générateur de gaz par une réaction chimique (préférentiellement une réaction acido-basique) libérant un gaz.
Il s'agit avantageusement d'un acide, d'un sel de polyacide partiellement salifié ou bien d'une solution tampon d'acide faible et de sa base conjuguée, ou d'un mélange de tels composés.
Selon l'invention le tampon acide/base dudit acide peut être tout tampon acide /base de l'acide faible comme par exemple un tampon acide citrique/citrate de sodium ou encore un tampon acide tartrique/tartrate de sodium. De préférence, nous citerons les alpha-hydroxy acides qui sont des acides faibles ayant préférentiellement un pKa compris entre 2 et 6 tel que l'acide citrique, l'acide tartrique, l'acide malique, l'acide lactique mais aussi l'acide phosphorique et l'acide pyrophosphorique et leurs sels éventuellement partiellement salifiés tels que le disodium pyrophosphate ou le sodium dihydrogénophosphate appelé encore phosphate monosodique.
Préférentiellement selon l'invention, l'agent activateur de gaz est choisi parmi un tampon acide tartrique/sel de tartrate (par exemple tartrate de sodium) ; un tampon acide citrique/citrate de sodium seul ; l'acide phosphorique, le phosphate de monosodium, le disodium pyrophosphate seuls ou en mélange avec un tampon acide citrique/ citrate de sodium.
Selon un mode de réalisation très préféré, l'agent activateur de gaz est un tampon acide citrique/ citrate de sodium seul ou en mélange avec du phosphate monosodique et/ou du disodium pyrophosphate.
Dans des compositions pour peaux sensibles ou pour peaux lésées comme les peaux acnéiques, la teneur en acide citrique/ citrate de sodium est de préférence inférieure ou égale à 2,4% par rapport au poids total de la composition intermédiaire A, de manière à limiter tout risque de picotement. Afin d'améliorer la tolérance et d'éviter la sensation de picotement, de manière préférée, le tampon acide citrique/citrate de sodium est employé en mélange avec le disodium pyrophosphate ou le sodium dihydrogénophosphate.
Selon l'invention, ledit agent activateur de gaz peut être présent dans la composition intermédiaire A en une quantité pouvant aller de 0,001% à 95% en poids par rapport au poids total de la composition intermédiaire A.

AGENT GENERATEUR DE GAZ :

Par agent générateur de gaz, on entend tout agent qui possède la propriété de générer par réaction chimique un gaz. On citera à cet égard tout composé qui, lorsqu'il est mélangé à un acide faible, peut former un gaz par une réaction chimique équivalente à la suivante :

$$NaHCO_3 + RCOOH \rightarrow RCOONa + H_2O + CO_2$$

Selon l'invention, le gaz généré à partir de l'agent générateur de gaz présent dans la composition intermédiaire B est de préférence du gaz carbonique ou du dioxyde de carbone ($CO_2$), et plus préférentiellement du dioxyde de carbone ($CO_2$).
Selon l'invention, l'agent générateur de gaz est de préférence choisi parmi le bicarbonate de sodium, le bicarbonate de potassium, le carbonate de sodium, le carbonate de potassium et leurs mélanges.
Préférentiellement selon l'invention, la formule intermédiaire B comprend un agent générateur de gaz carbonique qui de manière particulièrement préférée est du bicarbonate de sodium.
Ledit agent générateur de gaz peut être présent dans la composition B en une quantité allant de 1% à10%, préférentiellement de 2% à 8% en poids par rapport au poids de la composition intermédiaire B.
Selon l'invention, la formule intermédiaire A peut présenter un pH acide, avantageusement compris entre 1.0 et 6.0, et la formule intermédiaire B peut présenter un pH basique, avantageusement compris entre 7 et 12.
Selon l'invention, l'une (ou les) formule(s) intermédiaire (s), comprend de la brimonidine, telle quelle ou sous forme de sel, en une quantité correspondant à 0,01 à 1% en poids de la molécule brimonidine (c'est-à-dire, la (5-bromo-quinoxalin-6-yl)-(4,5-dihydro-1H-imidazol-2-yl)-amine),par rapport au poids de la composition totale.
De manière préférée, la composition totale (mélange de l'intermédiaire de formulation A avec l'intermédiaire

de formulation B) contient la brimonidine telle quelle ou sous forme de sel, en une quantité correspondant à 0,05% à 0,75% en poids, préférentiellement de 0,09% à 0,6% en poids, et plus préférentiellement de 0,1% à 0,5% en poids de la molécule brimonidine, par rapport au poids de la composition totale.

Dans la présente description, on entend par composition totale ou formule totale la composition du produit sous forme de mousse après le mélange desdites compositions intermédiaires. De préférence, la brimonidine est contenue dans la composition intermédiaire A qui présente une meilleure compatibilité avec l'actif.

De préférence, la brimonidine est employée sous forme de tartrate de brimonidine.

La formule intermédiaire A, peut se présenter sous toutes les formes galéniques compatibles avec la forme galénique désirée pour la composition finale obtenue par mélange de la formule A avec la formule B. Avantageusement la formule A peut-être un gel, une solution, une suspension, une émulsion (crème, crème sans tensioactif, lotion, lait, crème fluide), de préférence un gel ou émulsion contenant une phase aqueuse dans laquelle la brimonidine est solubilisée.

La formule intermédiaire B peut se présenter sous toutes les formes galéniques compatibles avec la forme galénique désirée pour la composition finale obtenue par mélange de la formule B avec la formule A. Avantageusement la formule B peut être un gel, une solution, une suspension, une émulsion (crème, crème sans tensioactif, lotion lait, crème fluide), de préférence une émulsion.

Selon un mode de réalisation de l'invention, l'une des deux formules intermédiaires (ie,la formule intermédiaire A ou la formule intermédiaire B) se présente sous forme d'un gel. Dans ce mode de réalisation, de préférence l'autre formule intermédiaire n'est pas sous forme de gel.

Chaque formule intermédiaire du kit ou du contenant à plusieurs compartiments tel que défini précédemment, conforme à l'invention comprend un milieu physiologiquement acceptable, véhiculant le ou les composés, et choisi de telle sorte que les composés soient aptes à réagir l'un avec l'autre pour former une composition auto-moussante lors du mélange d'au moins les formules intermédiaires A et B.

Ainsi, le mélange extemporané d'au moins deux formules, par exemple la formule A et la formule B, crée la composition sous forme de mousse selon l'invention.

Lors du mélange des deux formules A et B, l'agent générateur de gaz tel que le bicarbonate de sodium, réagit avec l'agent activateur de gaz tel que l'acide, et donne ainsi notamment le sel correspondant à l'acide, de l'eau et le gaz $CO_2$. C'est ce gaz, emprisonné dans les bulles de la composition, qui crée la mousse qui caractérise la composition auto-moussante de l'invention.

Ainsi par le mélange d'au moins la formule intermédiaire A et la formule intermédiaire B, on obtient la composition mousse dite composition totale selon l'invention.

Dans la composition obtenue après mélange d'au moins les formules A et B, il peut bien entendu rester de l'agent activateur de gaz et/ou de l'agent générateur de gaz n'ayant pas réagi.

Avantageusement, le kit ou le contenant unique à plusieurs compartiments selon l'invention peut être conçu de telle sorte que lors de la préparation de la composition selon l'invention, les formules intermédiaires A et B peuvent être mélangées en un rapport en poids A/B allant de 0,5 à 2, préférentiellement de 0,5 à 1,5, plus préférentiellement proche de 1 (c'est-à-dire de 0,9 à 1,1), et encore plus préférentiellement de 1. Cela signifie que le kit peut être conçu pour libérer simultanément des doses (en poids) des compositions intermédiaires A et B pouvant être en un rapport en poids allant de 2 doses de B pour 1 dose de A à 2 doses de A pour 1 dose de B, de préférence de 2 doses de B pour 1 dose de A à 3 doses de A pour 2 doses de B. Selon un mode de réalisation préféré de l'invention, le kit est conçu pour libérer simultanément 1 dose en poids de A et 1 dose en poids de B.

Selon l'invention le kit peut se présenter sous toute forme compatible avec d'une part une conservation séparée des formules intermédiaires A et B et d'autre part la capacité à réaliser extemporanément le mélange de A et de B.

Par exemple les formules intermédiaires A et B peuvent être conditionnées dans un boitier avec au moins deux compartiments séparés, chacun contenant A ou B.

Selon un autre aspect, le kit peut se présenter sous la forme d'une seringue à au moins deux corps séparés, chacun muni d'un piston, lesdits deux corps contenant les formules respectives A et B, et étant conçus pour libérer simultanément par exercice d'une force sur le piston les doses désirées des formules A et B.

L'invention concerne également un procédé de préparation d'une composition selon l'invention, caractérisé en ce que pour obtenir la composition sous forme de mousse, on mélange de façon extemporanée une formule intermédiaire A et une formule intermédiaire B du kit telles que définies plus haut, dans des proportions relatives en poids A/B pouvant aller de 0,5 à 2, préférentiellement de 0,5 à 1,5 et plus préférentiellement de 1.

Afin d'obtenir une mousse (composition finale) optimale, les inventeurs ont cherché expérimentalement les teneurs optimales en agent générateur de gaz (de préférence le bicarbonate de sodium) et en agent activateur de gaz (de préférence l'acide citrique et/ou le disodium pyrophosphate et/ou le sodium dihydrogénophosphate ou phosphate monosodique).

Ainsi, il a été déterminé expérimentalement que lorsque l'agent activateur de gaz est l'acide citrique, le ratio en poids acide citrique/bicarbonate de sodium est avantageusement compris entre 0,1 et 2 préférentiellement entre 0,5

à 1 et de façon très préférée égal à 0,7.
De la même façon, il a été déterminé que lorsque l'agent activateur de gaz est le disodium pyrophosphate, le ratio en poids disodium pyrophosphate/bicarbonate de sodium est compris entre 0,5 et 5 préférentiellement entre 1 et 3 et de façon très préférée est égal à 2,4.
De la même façon, il a été déterminé que lorsque l'agent activateur de gaz est le sodium dihydrogénophosphate, le ratio en poids sodium dihydrogénophosphate mono hydrate/bicarbonate de sodium est compris entre 0,5 et 5 préférentiellement entre 1 et 3 et de façon très préférée est égal à 2.
L'exemplification des ratios bicarbonate de sodium/acide citrique, bicarbonate de sodium/pyrophosphate de sodium, bicarbonate de sodium/sodium hydrogénophosphate est décrit dans l'exemple 4.

De façon surprenante, l'association acide citrique/citrate de sodium, disodium pyrophosphate ou sodium dihydrogenophosphate et un système gélifiant compatible avec la forme galénique a permis d'obtenir une formule aux propriétés physico-chimiques très stables et dans laquelle la brimonidine est particulièrement stable ne générant aucune sensation désagréable sur la peau et permettant la libération de gaz et ainsi la création de mousse.

L'exemple 2B ci-dessous montre que les compositions selon la présente invention possèdent une excellente stabilité tant physique que chimique.

Une composition est considérée stable physiquement lorsque ses caractéristiques organoleptiques, son pH, sa viscosité et l'homogénéité de la brimonidine n'évoluent pas avec le temps dans différentes conditions de températures : température ambiante (RT, ou TA), 30°C et 40°C.
Selon l'invention, la température ambiante correspond à une température allant de 15°C à 25°C

Une composition est considérée stable chimiquement lorsque la teneur en principe actif qu'elle contient n'évolue pas avec le temps dans les différentes conditions de températures (RT et 40°C)

Selon l'invention, la composition est considérée stable quand la teneur en brimonidine (exprimée en poids par rapport au poids de la formule intermédiaire) et mesurée par toute techniques et notamment HPLC, est incluse dans les spécifications allant de 90% à 110%.

La composition selon l'invention peut comprendre en outre un ou plusieurs agents choisis parmi les agents dispersants, les agents solubilisants, les agents stabilisants, les agents conservateurs, les corps gras, les agents épaississants, les colorants, les parfums, les tensioactifs, les agents gélifiants, les agents complexants, les agents neutralisants, les agents émulsionnants non moussants, les charges, les agents séquestrants, les agents réducteurs, les masques d'odeur, les agents plastifiants, les agents adoucissants, les agents hydratants, les pigments, les argiles, les charges minérales, les colloïdes minéraux, les polymères, les protéines, les agents nacrants, les cires, les huiles comme par exemple les paraffines,les silicones, des acides gras, des esters solides d'alcool gras ou d'acides gras, des gommes, les agents mouillants.

Des colorants hydrosolubles tels que le FD&C Blue 1 (de formule brute $C_{37}H_{34}N_2Na_2O_9S_3$) et des colorants liposolubles tel que le Rouge Soudan III ou le Red Nil présentent l'avantage de colorer un des intermédiaires de formulation. Cette coloration permet le contrôle du bon mélange des deux intermédiaires de formulation et de mettre en valeur la formation de la mousse. Cette coloration est notamment présentée dans les exemples et en figure 1.

AGENTS GELIFIANTS DE LA FORMULE INTERMEDIAIRE CONTENANT L'ACTIVATEUR DE GAZ

La composition intermédiaire A contenant au moins un agent activateur de gaz contient au moins un agent gélifiant et/ou agent de suspension tel que défini dans la revendication 1.

La formulation A peut contenir des fortes quantités d'acide et d'électrolytes, ces gels sont connus pour être très compliqués à stabiliser. La viscosité et le pouvoir suspensif de ces formules sont souvent durs à garantir dans le temps.

A titre d'exemple de gélifiants et/ou agents de suspension résistants à la fois au aux électrolytes et aux pH acides pouvant entrer dans les compositions A selon l'invention, on peut citer les mélanges prêt à l'emploi tels que le mélange Ammonium Acrylate / Acrylamide Copolymer & Polyisobutene & Polysorbate 20 vendu par Seppic sous le nom Sepiplus 265®, le Acrylamide / Sodium Acryloyldimethyl Taurate Copolymer & Isohexadecane & Polysorbate 80 vendu par Seppic sous le nom Simulgel 600 PHA®, le Polyacrylate-13 & Polyisobutene & Polysorbate 20 vendus par Seppic sous le nom Sepiplus 400®, Acrylates/C10-30 Alkyl Acrylate Crosspolymer vendu par la société Lubrizol sous les noms Pemulen™ TR-1 Polymeric Emulsifier et Pemulen™ TR-2 Polymeric Emulsifierles polysaccharides avec à titre d'exemples non limitatifs la gomme de xanthane telle que le Xantural180® vendu par la société Kelco, la gellan gum vendu sous le nom de Kelcogel® par la société Kelco, la Sclerotium Gum vendu sous le nom Amigel® par Alban Muller industrie, la gomme guar et ses dérivés tel que Hydroxypropyl Guar vendu sous le nom Jaguar HP-105® revendu par Rodhia, la cellulose et ses dérivés tel que la microcrystalline cellulose et carboxymethyl cellulose de sodium vendue sous le nom Blanose CMC 7H4XF® par la société Hercules, l'hydroxypropylmethylcellulose en particulier le produit vendu sous le nom de Methocel E4M® premium par la société Dow Chemical ou l'hydroxyéthylcellulose, en particulier, le produit vendu sous le nom de Natrosol HHX 250® par la société Aqualon, la famille des aluminium magnésium silicates tel que le

Veegum K®, Veegum Plus® ou le Veegum Ultra® vendu par la société Vanderbilt, la bentonite vendue sous le nom de polargel HV®, la famille des amidons modifiés tels que l'amidon de pomme de terre modifié vendu sous le nom de Structure Solanace®, la famille des carraghénanes en particulier réparties sous quatre grandes familles : κ, λ, β, ω tel que les Viscarin® et les Gelcarin® commercialisés par la société IMCD. Ou encore la Polyvinyl Alcohol connue aussi sous l'abréviation PVA revendu par Merck sous le nom POLYVINYL ALCOHOL 40-88®. De façon préférée, le Vegum K®, le Simulgel 600 PHA® et le Xantural 180® seront utilisés seul ou en association deux à deux ou les trois ensemble.

L'agent gélifiant tel que décrit ci-dessus peut être utilisé aux concentrations préférentielles allant de 0,001% à 15 % et, plus préférentiellement, allant de 0,15% à 5% en poids par rapport au poids de la formule intermédiaire A.

AGENTS GELIFIANTS DE LA FORMULE INTERMEDIAIRE CONTENANT LE GENERATEUR DE GAZ

A titre d'exemple de gélifiants et/ou agent de suspension et/ou gélifiants résistants à la fois aux électrolytes et aux pH basiques pouvant entrer dans les compositions intermédiaires B selon l'invention, on peut citer les polymères d'acide acryliques comme l'Acrylates/C10-30 Alkyl Acrylate Crosspolymer tel que les carbomers dits non sensibles aux électrolytes, vendus sous le nom d'Ultrez 20®, d'Ultrez 10®, de Carbopol 1382® ou de Carbopol ETD2020NF®, AQUA SF1® vendus par la Société Lubrizol, le mélange Ammonium Acrylate / Acrylamide Copolymer & Polyisobutene & Polysorbate 20 vendu par Seppic sous le nom Sepiplus 265®, le Acrylamide / Sodium Acryloyldimethyl Taurate Copolymer & Isohexadecane & Polysorbate 80 vendu par Seppic sous le nom Simulgel 600 PHA®, le Polyacrylate-13 & Polyisobutene & Polysorbate 20 vendus par Seppic sous le nom Sepiplus 400®, Acrylates/C10-30 Alkyl Acrylate Crosspolymer vendu par la société Lubrizol sous les noms Pemulen™ TR-1 Polymeric Emulsifier et Pemulen™ TR-2 Polymeric Emulsifier, les polysaccharides avec à titre d'exemples non limitatifs la gomme de xanthane telle que le Xantural180® vendu par la société Kelco, la gellan gum vendu sous le nom de Kelcogel® par la société Kelco, la Sclerotium Gum vendu sous le nom Amigel® par Alban Muller industrie, la gomme guar et ses dérivés tel que Hydroxypropyl Guar vendu sous le nom Jaguar HP-105® revendu par Rodhia, la cellulose et ses dérivés tel que la microcrystalline cellulose et carboxymethyl cellulose de sodium vendue sous le nom Blanose CMC 7H4XF® par la société Hercules, l'hydroxypropylmethylcellulose en particulier le produit vendu sous le nom de Methocel E4M® premium par la société Dow Chemical ou l'hydroxyéthylcellulose, en particulier, le produit vendu sous le nom de Natrosol HHX 250® par la société Aqualon, la bentonite vendue sous le nom de polargel HV®, la famille des aluminium magnésium silicates tel que le Veegum K®, Veegum Plus® ou le Veegum Ultra® vendu par la société Vanderbilt, , la famille des amidons modifiés tels que l'amidon de pomme de terre modifié vendu sous le nom de Structure Solanace® ou de Farine de Tapioca connue sous le nom de Naviance Tapioca P® revendu par Akzo Novel ou de la famille des carraghénanes en particulier réparties sous quatre grandes familles : κ, λ, β, ω tel que les Viscarin® et les Gelcarin® commercialisés par la société IMCD. De façon préférée, le Vegum K®, le Simulgel 600 PHA® et le Xantural 180® seront utilisés seul ou en association deux à deux ou les trois ensemble

L'agent gélifiant tel que décrit ci-dessus peut être utilisé aux concentrations préférentielles allant de 0,001% à 15 % et, plus préférentiellement, allant de 0,15% à 5% en poids par rapport au poids de la formule intermédiaire B.

AGENTS HUMECTANTS

Parmi les agents humectants et/ou émollients qui ont pour rôle d'hydrater la peau et de faciliter l'application de la formulation, on utilise optionnellement, sans que cette liste soit limitative, des composés tels qu'un polyol miscible à l'eau à la température ambiante (25 °C) notamment choisi parmi les polyols ayant notamment de 2 à 20 atomes de carbones, de préférence ayant de 2 à 10 atomes de carbone, et préférentiellement ayant de 2 à 6 atomes de carbones, tels que le glycérol, les dérivés de glycol tel que le propylène glycol, le butylène glycol, le pentylène glycol, l'hexylène glycol, le dipropylène glycol, le diéthylène glycol et leurs mélanges mais aussi les sucres (à titre d'exemple le glucose, le lactose), les polyethylene glycol (PEG) (à titre d'exemple le Lutrol E400®), l'urée, les acides aminés (à titre d'exemple la sérine, la citrulline, l'arginine, l'asparagine, l'alanine).

A titre d'agent humectant et/ou émollient préféré, on peut citer la glycérine et le propylène glycol.

Les agents humectants peuvent être utilisés, seuls ou en association, aux concentrations préférentielles allant de 0,001% à 30 % et, plus préférentiellement, allant de 0.01% à 10% en poids par rapport au poids de la formule totale.

AGENTS CHELATANTS

Parmi les agents chélatants, on peut citer à titre d'exemples non limitatifs l'acide éthylène diamine tétra-acétique (EDTA), l'acide diéthylène triamine penta-acétique (DTPA), l'acide éthylène diamine-di (O-hydroxyphényl acétique) (EDDHA), l'acide hydroxy-2-éthylène diamine triacétique (HEDTA), l'acide éthyldiamine-di (O-hydroxy-p-méthyl phényl) acétique (EDDHMA) et l'acide éthylène diamine-di (5-carboxy-2-hydroxyphényl) acétique (EDDCHA). A titre d'agent chélatant préféré, on peut citer l'acide éthylène diamine tétra-acétique (EDTA) vendu notamment sous le nom Titriplex

III® il peut être utilisé aux concentrations préférentielles allant de 0,001% à 1 % et, plus préférentiellement de 0.05% à 0.1% en poids par rapport au poids de la formule totale.

EXCIPIENTS AUX PROPRIETES COMPLEMENTAIRES

La composition selon l'invention peut contenir un ou plusieurs excipients aux propriétés spécifiques comme par exemple, à titre non limitatif, l'allantoine aux propriétés anti-irritantes, le dipotassium glycyrrhizate pour ses propriétés anti-inflammatoires ou encore l'alpha bisabolol cicatrisant, le lithium digluconate pour ses propriétés anti-rougeurs.

CHARGES ET PARTICULES

Des charges et/ou des particules peuvent être utilisées pour stabiliser et booster la mousse. Certaines d'entre elles ont la propriété particulière de se placer à l'interface eau/air et ainsi stabiliser cette interface. Comme charges, on peut citer le talc, les oxydes de métaux tel que l'oxyde de zinc, le dioxyde de titane $TiO_2$ T2000 vendu par la société Merck sous le nom Eusolex® T-2000, les argiles tel que les laponites, bentones ou les bentonites mais aussi les ethers de cellulose tel que le Methocel® K100 LV commercialisé par la société DOW, les silices telles que l'Aerosil® R972 vendue par la société EVONIK ou la SILICE HDK® H13L vendue par WACKER elles peuvent être utilisées aux concentrations allant de 0.01% à 10% en poids par rapport au poids de la formule totale.

LES HUILES DE LA PHASE GRASSE

La composition selon l'invention peut également comprendre une phase grasse. Cette phase grasse peut être présente dans l'une et/ou l'autre des compositions intermédiaires A et B. La phase grasse peut selon la forme galénique des formules intermédiaires représenter de 0% à 95% en poids par rapport au poids de chaque formule intermédiaire.

La phase grasse de la composition selon l'invention peut comprendre par exemple, les huiles végétales, minérales, animales ou synthétiques, des huiles de silicones, et leurs mélanges

Comme exemple d'huile minérale, on peut citer par exemple des huiles de paraffine de différentes viscosités telles que le Primol 352®, le Marcol 82®, Marcol 152® vendus par la société Esso.

Comme huile végétale, on peut citer l'huile d'amande douce, l'huile de palme, l'huile de soja, l'huile de sésame, l'huile de tournesol, l'huile d'olive.

Comme huile animale ou leur substitut d'origine végétale, on peut citer la lanoline, le squalene, l'huile de poisson, avec comme dérivé le perhydrosqualene vendu sous le nom Sophiderm® par la société Sophim.

Comme huile synthétique, on peut citer un ester tel que le cetearyl isononanoate comme le produit vendu sous le nom de Cetiol SN PH® par la société Cognis France, l'isononyl isononanoate comme le DUB ININ® revendu par les Stearine Dubois, le diisopropyl adipate comme le produit vendu sous le nom de Crodamol DA® par la société Croda, le palmitate d'isopropyle comme le produit vendu sous le nom de Crodamol IPP® par la société Croda, le caprylique caprique triglyceride tel que Miglyol 812® vendu par la société Univar. Comme polyisobutene hydrogéné, on peut citer les Parleam® vendus par la société Rossow.

Comme huile de silicone, on peut citer une dimethicone comme le produit vendu sous le nom de Q7-9120 Silicone Fluid® de viscosité de 20 cst à 12500 cst par la société Dow Corning, une cyclomethicone comme le produit vendu sous le nom de ST-Cyclomethicone 5NF® également par la société Dow Corning.

LES CORPS GRAS NON LIQUIDES

La composition selon l'invention, et en particulier la formule intermédiaire B, peut également comprendre des corps gras solides tel que des cires naturelles ou synthétiques, des acides gras tels que l'acide stéarique, des alcools gras tels que le Speziol C18® pharma ou le Speziol C16® vendu par la société Cognis, et des agents de texture de type tribehenate, tel que le Compritol 888® vendu par la société Gattefossé ou les huiles de ricin hydrogénées telles que le Cutina HR® vendu par la société Cognis ou le glyceryl stearate tel que le GELEOL® vendu par la société Gattefosse ou encore le Dc 9045 Elastomer Blend® vendu par la société Dow Corning.

Ces corps gras non liquides peuvent être utilisés seuls ou en mélange de 0 à 30% en poids par rapport au poids de la formule totale. Il a été cependant observé une qualité de mousse exceptionnelle lorsque des alcool gras de formule $CH_3(CH_2)_nOH$ (n est compris entre 11 et 23) sont présents à des teneurs supérieures à 1% en poids par rapport au poids de la formule totale.

EMULSIFIANTS NON IONIQUES

La composition selon l'invention et notamment la formule intermédiaire B peut également comprendre un ou

plusieurs émulsifiants non ioniques.

A titre d'émulsifiants préférés on peut citer des émulsifiants hydrophiles de type Glyceryl Stearate (and) PEG-100 Stearate vendu sous le nom Arlacel 165FL® par la société Uniquema, des émulsifiants lipophiles de type Glucate SS® et Glucamate SSE®, Polyoxyethylene (21) Stearyl Ether vendu sous le nom Brij721® par la société Uniquema ou encore dans la meme famille le Brij S2®, le Brij S20®. La self emulsifying Wax vendu par Croda sous le nom de Polawax NF®. On peut citer également des tensioactifs non ioniques présentant une HLB élevée, les esters de sorbitan tels que le POE(20) sorbitan monooléate, vendu sous le nom de « Tween 80® » (HLB=15) ; le POE(20) sorbitan monostéarate vendu sous le nom de « Tween 60® » (HLB=14.9) ; les éthers d'alcools gras tels que le POE (21) stéaryl ether (HLB= 15.5), ou le ceteareth 20 vendu sous le nom de Eumulgin B2 PH® par Cognis (HLB de 15,5, ou des tensioactifs non ioniques de bas HLB, les esters de sorbitan, tels que le monostéarate de sorbitan (vendu sous le nom de Span 60® par Uniquema), les esters de glycérol tels que le monostéarate de glycérol (Cutina GMS® de chez Cognis), les esters de saccharose de bas HLB comme le distéarate de saccharose. Dans un autre mode selon l'invention les tensioactifs utilisables sont les esters de polyglycerol. Il s'agit d'esters d'acides gras polyglycerinés obtenus par condensation de glycerine. Des émulsionnants glycolipidique tel que le Montanov 202® vendu par la société SEPPIC. Certains émulsionnants peuvent être vendus sous forme de mélange tel que les Emulium Kappa® et Emulium Delta® vendu par Gattefosse. Ces Tensio actifs peuvent être utilisés seuls ou en association de façon à ce que le HLB du système soit supérieur à 12 et préférentiellement supérieur à 15.

De tels émulsifiants peuvent être utilisés entre 0.01% et 30 % en poids par rapport au poids de la composition totale, préférentiellement entre 0.1% et 15%, et plus préférentiellement entre 0.5% et 7 %.

AGENTS CONSERVATEURS:

On peut citer à titre d'exemple de conservateurs, le chlorure de benzalkonium, le bronopol, la chlorhexidine, le chlorocrésol et ses dérivés, l'alcool éthylique, le phénoxyéthanol, le sorbate de potassium, la diazolidinylurée, l'alcool benzylique, les parabens, le benzoate de sodium ou leurs mélanges.
Au titre de système conservateur préféré on peut citer l'association phenoxyethanol et pentylene glycol.

Les exemples suivants illustrent l'invention sans en restreindre la portée.

EXEMPLES

EXEMPLE 1: exemples de formules

Exemples de Formules A : Compositions intermédiaires A contenant l'agent activateur de gaz:

Les formules intermédiaires A ont été préparées suivant le procédé suivant :

Etape 1 : A une température supérieure à 60°C, Ajouter sous agitation le gelifiant principal (Veegum K) à la phase d'eau principale.
Etape 2 : Ajouter les agents tampons acide puis refroidir à 40°C
Etape 3 : Sous agitation magnétique solubiliser la brimonidine dans la phase aqueuse annexe restante. Transvaser cette phase annexe dans la phase principale
Etape 4 : Ajouter le/les autre(s) gélifiant(s) sous agitation
Etape 5 : Ajouter les autres excipients de la formule sous agitation

Dans les exemples de formules ci-dessous, les quantités sont exprimées par rapport au poids de la formule intermédiaire et non par rapport au poids de la formule totale.

Exemple A1 :

| Dénomination INCI | % en poids |
|---|---|
| EAU | QSP 100 |
| DISODIUM EDTA | 0.1 |
| MAGNESIUM ALUMINIUM SILICATE | 2.5 |

(suite)

| Dénomination INCI | % en poids |
|---|---|
| GOMME DE XANTHANE | 1.5 |
| ACIDE CITRIQUE | 1.4 |
| CITRATE DE SODIUM | 1 |
| DISODIUM PYROPHOSPHATE | 7.2 |
| POLOXAMER 124 | 0.2 |
| PROPYLENE GLYCOL | 4 |
| BENZOATE DE SODIUM | 0.2 |
| TARTRATE DE BRIMONIDINE | 1 |

Exemple A2 :

| INCI Name | % |
|---|---|
| EAU | QSP 100 |
| DISODIUM EDTA | 0.1 |
| MAGNESIUM ALUMINIUM SILICATE | 2.5 |
| GOMME DE XANTHANE | 1.5 |
| ACIDE CITRIQUE | 3.5 |
| CITRATE DE SODIUM | 2.7 |
| PROPYLENE GLYCOL | 4 |
| BENZOATE DE SODIUM | 0.2 |
| TARTRATE DE BRIMONIDINE | 1 |

Exemple A3 :

| Dénomination INCI | % en poids |
|---|---|
| EAU | QSP 100 |
| DISODIUM EDTA | 0.1 |
| MAGNESIUM ALUMINIUM SILICATE | 2.5 |
| GOMME DE XANTHANE | 1.5 |
| ACIDE CITRIQUE | 1.5 |
| CITRATE DE SODIUM | 0.5 |
| SODIUM DIHYDROGENOPHOSPHATE | 6.2 |
| PROPYLENE GLYCOL | 4 |
| BENZOATE DE SODIUM | 0.2 |
| TARTRATE DE BRIMONIDINE | 1 |

Exemple A4

| Dénomination INCI | % en poids |
|---|---|
| WATER | QSP 100 |
| DISODIUM EDTA | 0.1 |
| MAGNESIUM ALUMINIUM SILICATE | 2.5 |
| XANTHAN GUM | 1.5 |
| CITRIC ACID | 1.5 |
| SODIUM CITRATE | 0.5 |
| SODIUM DIHYDROGENOPHOSPHATE | 6.2 |
| PROPYLENE GLYCOL | 4 |
| SODIUM BENZOATE | 0.2 |
| BRIMONIDINE TARTRATE | 0.36 |

Exemple A5 :

| Dénomination INCI | % en poids |
|---|---|
| EAU | QSP 100 |
| DISODIUM EDTA | 0.1 |
| GOMME DE XANTHANE | 0,7 |
| MAGNESIUM ALUMINIUM SILICATE | 2.5 |
| DISODIUM PYROPHOSPHATE | 6 |
| ACIDE CITRIQUE | 1.8 |
| CITRATE DE SODIUM | 1.3 |
| POLOXAMER 124 | 0,2 |
| TARTRATE DE BRIMONIDINE | 1 |
| PROPYLENE GLYCOL | 4,0 |

Exemple A7 :

| Dénomination INCI | % en poids |
|---|---|
| EAU | QSP 100 |
| DISODIUM EDTA | 0.1 |
| GOMME DE XANTHANE | 0,7 |
| MAGNESIUM ALUMINIUM SILICATE | 2.5 |
| BENZOATE DE SODIUM | 0.2 |
| DISODIUM PYROPHOSPHATE | 7.2 |
| ACIDE CITRIQUE | 1.4 |

(suite)

| Dénomination INCI | % en poids |
|---|---|
| CITRATE DE SODIUM | 1 |
| POLOXAMER 124 | 0,2 |
| TARTRATE de BRIMONIDINE | 1 |
| PROPYLENE GLYCOL | 4,0 |

Formules B : Compositions intermédiaires B contenant l'agent générateur de gaz:

Les formules intermédiaires B ont été préparées suivant le procédé suivant :

Etape 1' : A une température supérieure à 60°C, ajouter sous agitation les gélifiants à la phase d'eau principale.

Etape 2' optionnel : Parallèlement chauffer la phase grasse (contenant les huiles, les cires, et les tensio-actifs) à une température supérieure à 60°C.

Etape 3' I : A une température supérieure à 60°c réaliser l'émulsion en ajoutant la phase grasse à la phase principale.

Etape 4' : Ajouter les additifs tel que les conservateurs ou de l'éthanol à une température adaptée à l'additif.

Etape 5' : Neutraliser le mélange.

Etape 6' : A une température inférieure à 40°C ajouter le bicarbonate de sodium

Exemple B1

| Dénomination INCI | % en poids |
|---|---|
| EAU | QSP 100 |
| MAGNESIUM ALUMINIUM SILICATE | 2.5 |
| GOMME DE XANTHANE | 0.6 |
| POLYSORBATE 80 | 0.8 |
| STEARETH-20 | 2.8 |
| ALCOOL CETOSTEARYLIQUE | 1.5 |
| HUILE MINERALE | 8 |
| TRIETHANOLAMINE | 1.2 |
| HYDROGENO CARBONATE DE SODIUM | 5 |

Exemples B2 et B3 :

| Dénomination INCI | B2 % en poids | B3 % en poids |
|---|---|---|
| EAU | QSP 100 | QSP 100 |
| SODIUM CARBOXYMETHYLCELLULOSE | 0.5 | 0.2 |
| HYDROXYETHYLCELLULOSE | 0.25 | 0.1 |
| STEARETH-20 | 1.8 | 1.8 |

(suite)

| Dénomination INCI | B2 % en poids | B3 % en poids |
|---|---|---|
| STEARATE DE GLYCERYLE (ET) STEARATE DE PEG-100 | 2.7 | 2.7 |
| ALCOOL CETOSTEARYLIQUE | 1 | 7 |
| POLYISOBUTENE HYDROGENE | 9 | 9 |
| TRIETHANOLAMINE | 1.2 | 1.2 |
| PROPYLENE GLYCOL | 5 | 5 |
| PHENOXYETHANOL | 1 | 1 |
| HYDROGENO CARBONATE DE SODIUM | 3 | 3 |

Exemple B5:

| Dénomination INCI | % en poids |
|---|---|
| EAU | QSP 100 |
| MAGNESIUM ALUMINIUM SILICATE | 3.5 |
| GOMME DE XANTHANE | 1 |
| POLYSORBATE 80 | 2 |
| ACIDE STEARIQUE | 3 |
| ALCOOL CETOSTEARYLIQUE | 1.5 |
| POLYISOBUTENE HYDROGENE | 8 |
| TRIETHANOLAMINE | 1.8 |
| HYDROGENO CARBONATE DE SODIUM | 3 |
| PHENOXYETHANOL | 0.8 |

Exemple B6:

| Dénomination INCI | % en poids |
|---|---|
| EAU | QSP 100 |
| MAGNESIUM ALUMINIUM SILICATE | 2.5 |
| GOMME DE XANTHANE | 0.6 |
| CETEARETH-20 | 3 |
| TRIGLYCERIDE CAPRYLIQUE/CAPRIQUE | 6 |
| ALCOOL CETOSTEARYLIQUE | 7 |
| TRIETHANOLAMINE | 0.1 |
| HYDROGENO CARBONATE DE SODIUM | 5 |
| PHENOXYETHANOL | 0.8 |

Exemple B7:

| Dénomination INCI | % en poids |
|---|---|
| EAU | QSP 100 |
| MAGNESIUM ALUMINIUM SILICATE | 2.5 |
| GOMME DE XANTHANE | 0.5 |
| CETEARETH-20 | 3 |
| ALCOOL CETOSTEARYLIQUE | 3 |
| DIBEHENATE DE GLYCERYLE | 3 |
| TRIGLYCERIDE CAPRYLIQUE/CAPRIQUE | 6 |
| HYDROXYDE de SODIUM | 0.09 |
| PHENOXYETHANOL | 0.8 |
| HYDROGENO CARBONATE DE SODIUM | 5 |

Le tableau ci-dessous représente les mélanges dans un ratio pondéral 1:1 des compositions intermédiaires A et B décrites ci-avant. Une croix à l'intersection de deux intermédiaires de formulation indique que le mélange a été testé et a généré une mousse aux propriétés recherchées.

| Formule B / Formule A | B1 | B2 | B3 | B5 | B6 | B7 |
|---|---|---|---|---|---|---|
| A1 | X | X | X | X | X | X |
| A2 | X | X | X | X | X | X |
| A3 | X | X | X | X | X | X |
| A5 | X | X | X | X | X | X |
| A4 | X | X | X | X | X | X |
| A7 | X | X | X | X | X | X |

EXEMPLE 2A : mesures de densité de mousse

A partir des exemples de formules décrites dans l'exemple 1, des mesures de densité de mousses ont été réalisées au moment de la mise en contact des 2 formules intermédiaires A et B (T0) puis lorsque la réaction chimique générée par la mise en contact des deux compositions est terminée :

Densité formule A5 placebo, c'est-à-dire sans brimonidine (mais avec colorant bleu) = 1.108
Densité formule B7 = 1.021
Mousse A5/B7 (50/50)= 0.290

La mesure de la densité de la mousse montre que le volume a été augmenté d'un facteur 4 et confirmé par les photos en Figure 1. La photo de gauche représente le moment du mélange (T0) et la photo de droite représente la mousse obtenue lorsque la réaction chimique acide/ base est complète.

EXEMPLE 2B : stabilité

Les tableaux la et Ib ci-dessous rassemblent les données de stabilité physique des formules intermédiaires A 1 et A 3 décrites dans l'exemple 1, contenant de la brimonidine.

| Formule A1 | T0 | | T1 Mois | T2Mois | T3Mois | T6Mois |
|---|---|---|---|---|---|---|
| pH | 3.99 | TA | 4.11 | 4.15 | 4.18 | 4.06 |
| | | 5°C | 4.02 | 4.09 | - | - |
| | | 40°C | 4.16 | 4.17 | - | 4.11 |
| Viscosité cP brookfield RV DVII Aig5vit50 | 6200 | TA | 6020 | 5880 | - | 6900 |
| | | 40°C | 5600 | 5710 | - | 5600 |

| Formule A3 | T0 | | T1 Mois | T2Mois | T3Mois | T6Mois |
|---|---|---|---|---|---|---|
| pH | 3.46 | TA | 3.83 | 3.85 | 3.84 | 3.87 |
| | | 5°C | 3.69 | 3.67 | 3.64 | 4.15 |
| | | 40°C | 3.98 | 3.91 | 3.93 | 3.99 |
| Viscosité cP brookfield LV DVII Aig63vit20 | 14219 | TA | 13027 | 18877 | 11600 | 10648 |
| | | 5°C | 13400 | 14150 | 11338 | 10708 |
| | | 40°C | 14999 | 13407 | 12567 | 10588 |

Le tableau II ci-dessous détaille les données de stabilité chimique de la brimonidine dans la formule intermédiaire A 1.

| Formule A 1 | T0 | | T1 Mois | T2Mois | T3Mois |
|---|---|---|---|---|---|
| %p/ BRIMONIDINE (HPLC) | 97.4 | RT | 97.3 | 96.3 | 98.9 |
| | | 40°C | 97.5 | 96.2 | 99.2 |

EXEMPLE 3 : Etude comparative de mesure de l'irritation

Protocole d'étude.

L'étude est réalisée selon le protocole TG439 OCDE en vigueur pour le temps d'application court (temps de contact RHE/produit 15min). Ce protocole est adapté pour un temps d'application long (temps de contact RHE/produit 18h).

L'objectif de cette étude est d'évaluer la tolérance des supports des formules complètes et intermédiaires sur épidermes humains reconstruits (RHE, modèle Episkin) au travers de :

- l'évaluation de la réduction du MTT (viabilité cellulaire)
- la mesure de la libération d'IL-1alpha (marqueur d'irritation)

Les formules testées sont :

- Une composition intermédiaire de formulation acide: exemple A7 placebo (c'est à dire, ne contenant pas de brimonidine),
- Une composition intermédiaire de formulation basique: exemple B7,

- La formule complète composée du mélange: A7 placebo + B7 (dans un ratio 50/50 en poids),
- Une référence commerciale sous forme de crème.

Résultats de l'étude :

| Mélange testé | Exposition courte | Exposition longue | Conclusion |
|---|---|---|---|
| | Viabilité (%) | Viabilité (%) | Potentiel irritant |
| B7 | 89.8 | 93.3 | Non irritant |
| A7 placebo | 86.0 | 84.5 | Non irritant |
| Formule complète | 95.8 | 83.4 | Non irritant |
| Ref commerciale | 99.8 | 91.1 | Non irritant |

| Mélange testé | Exposition courte | Exposition longue |
|---|---|---|
| | IL-1a vs contrôle | IL-1a vs contrôle |
| B7 | 1.5 | 2.0 |
| A7 placebo | 2.2 | 2.3 |
| Formule complète | 1.9 | 3.1 |
| Ref commerciale | 2.4 | 3.6 |

Les mesures du MTT selon le protocole OCDE en vigueur indiquent que toutes les formules testées sont non irritantes.
Le dosage d'IL-1a de la formule complète selon l'invention après un temps court et un temps long d'exposition montre un taux de marqueurs d'irritation plus bas qu'après application de la référence commerciale.

EXEMPLE 4 :

Il a été établit de façon empirique la teneur idéale en acide citrique, pyrophosphate de sodium et sodium dihydrogénophosphate monohydrate pour réagir avec 5% de bicarbonate de sodium. Les valeurs sont exprimées en pourcentage poids/poids par rapport au poids de chacune des deux formules intermédiaires.

| | Ratio1 | Ratio2 | Ratio3 |
|---|---|---|---|
| Bicarbonate de Sodium | 5% | 5% | 5% |
| Acide citrique | 3.5% | - | - |
| Disodium pyrophosphate | - | 12 | - |
| Sodium Dihydrogénophosphate monohydrate | | - | 7.2% |

Afin que le pH de la formule contenant l'activateur de gaz présente une compatibilité optimale avec la peau, nous avons ajouté du citrate de sodium afin de créer un tampon acide citrique/citrate de sodium.
Une partie du tampon l'acide citrique/citrate de sodium peut avantageusement être substituée par du disodium pyrophosphate et vice versa comme les teneurs citées à titre d'exemple dans le tableau ci-dessous :

Tableau III : les valeurs sont exprimées en pourcentage poids/poids par rapport au poids de chacune des deux formules intermédiaires

| | E 1 | E2 | E 3 | E 4 | E 5 | E 6 | E 7 |
|---|---|---|---|---|---|---|---|
| Bicarbonate de Sodium | 5% | 5% | 5% | 5% | 3% | 3% | 3% |
| Acide citrique | 3.5% | 1.75% | 1.4% | 0 | 2.1% | 1.05% | 0 |
| Citrate de Na | 2.7% | 1.3% | 1% | 0 | 1.6% | 1.15% | 0 |
| Disodium pyrophosphate | 0 | 6% | 7.2% | 12% | 0 | 3.6% | 7.2% |

Une partie du tampon l'acide citrique/citrate de sodium peut avantageusement être substituée par du sodium dihydrogénophosphate mono hydrate et vice versa comme les teneurs citées à titre d'exemple dans le tableau IV ci-dessous :

Tableau IV : les valeurs sont exprimées en pourcentage poids/poids par rapport au poids de chacune des deux formules intermédiaires

| | E 1 | E8 | E9 |
|---|---|---|---|
| Bicarbonate de Sodium | 5% | 5% | 5% |
| Acide citrique | 3.5% | 1.5% | 0 |
| Citrate de Na | 2.7% | 0.5% | 0 |
| Sodium Dihydrogénophosphate monohydrate | 0 | 6.2% | 10% |

Dans un mode particulier, il a été déterminé que lorsque la quantité d'acide citrique est supérieure ou égale à 1,4, la quantité de mousse est optimale lorsque le disodium pyrophosphate est présent dans la composition selon l'équation suivante :

-

$$[C]=2.4[B]-2.4[A]/0.7$$

Lorsque :

[C] = teneur en poids en disodium pyrophosphate dans la composition intermédiaire A
[A] = teneur en poids en acide citrique monohydrate dans la composition intermédiaire A
[B] = teneur en poids en bicarbonate de sodium dans la composition intermédiaire B

L'équation ci-dessus permet ainsi de calculer les teneurs optimales entre le bicarbonate de sodium l'acide citrique et le pyrophosphate de sodium.

EXEMPLE 5 : Etude comparative de profil de libération- perméation

Protocole de l'étude :

Les formules testées dans cette étude sont appliquées sur des peaux humaines entières excisées montées sur cellule de Franz à raison de 5mg/$cm^2$ à 32°C.
Après 16 heures d'application, le tartrate de brimonidine est dosé dans : la faction non absorbée, le stratum corneum, l'épiderme, le derme et le liquide récepteur.
Cette étude permet d'étudier l'influence de la formulation sur la libération du principe actif et sa perméation au travers de la peau. L'objectif est de comparer la répartition de la tartrate de brimonidine dans les différentes couches de la peau dans le cas de l'application d'une formule de référence et de l'application d'une composition sous forme de mousse chimique.
Les formules testées sont :

- Une composition commerciale de référence contenant 0.33% en poids de Brimonidine sous forme de base correspondant à 0.5% en poids de tartrate de brimonidine ;
- Une Référence sous forme de gel contenant 0.12% en poids de Brimonidine sous forme de base correspondant à 0.18% en poids de tartrate de brimonidine ;
- Une formule de mousse selon l'invention à 0.5% en poids de tartrate de brimonidine (soit 0.33% en poids de brimonidine) composée du mélange des compositions A 3 et B 7 de l'exemple 1 (dans un ratio 50/50 en poids),
- Une formule de mousse selon l'invention à 0.18% en poids de tartrate de brimonidine (soit 0.12% en poids de brimonidine) composée du mélange des compositions A 4 et B 7 de l'exemple 1 (dans un ratio 50/50 en poids).

Cellules de diffusion :

Les cellules de diffusion utilisées sont des cellules de diffusion statiques, sur la base de cellule de diffusion modèle de Franz, avec les caractéristiques suivantes:

- Domaine d'application = 2 $cm^2$
- Volume du compartiment de fluide récepteur = 3 ml

Le compartiment récepteur est entouré par une chemise d'eau chauffée à 37 ° C ± 1 ° C, assurant une température de 32 ° C ± 1 ° C à la surface de la peau. Le compartiment récepteur est séparé du compartiment donneur par la membrane de peau, la face épidermique étant du côté du donneur. Le compartiment Récepteur contenant un barreau d'agitation magnétique, a été remplis avec le fluide récepteur de manière à empêcher toute formation de bulles d'air. Durant le temps de diffusion, le fluide récepteur était agité en continu afin d'assurer l'homogénéisation

Préparation des échantillons de peau :

Des peaux abdominales issues de chirurgie esthétique ont été utilisées dans cette étude. Dès l'arrivée des échantillons, l'hypoderme a été séparé de l'ensemble à l'aide d'une pince à épiler et les restes ont été doucement lavés et stockés à plat dans une feuille d'aluminium pour la congélation à -20 ° C. Le jour de l'expérimentation les échantillons de peaux ont été décongelés puis coupés en morceaux pour être compatibles avec la géométrie de la cellule de diffusion. Les échantillons de peau issus de donneurs âgé de 42, 44 et 69 ans ont été montés sur la cellule de diffusion avec du PBS en tant que fluide récepteur. L'épaisseur moyenne de la peau était de 0,89 ± 0,07 mm avec un maximum de 1,39 mm et un minimum de 0,45 mm. Epaisseurs de tous les spécimens.

Après au moins 45 min à l'équilibre avec le fluide récepteur, l'intégrité de la peau est évaluée par la mesure trans-épidermique de perte insensible en eau (TEWL). Toutes les cellules où les mesures de TEWL en dehors des critères d'acceptation sont soigneusement nettoyées et laissées à l'équilibre pendant une période prolongée avant une nouvelle mesure de la TEWL. La Valeur moyenne TEWL était de 5,51 ± 1,63 g / $m^2$ / h.

Paramètres de l'étude:

Température ambiante: 21,7 ° C
Humidité relative: 45,6%

Résultats de l'étude :

Dans le stratum corneum et dans l'épiderme une différence de pénétration cutanée a été observée entre les formules mousse de l'invention à 0.18% de tartrate de brimonidine composée du mélange des compositions A 4 et B 7 (dans un ratio 50/50 en poids), par rapport à un gel de référence contenant 0.18% de tartrate de brimonidine) comme décrit dans la figure 2A.

Dans le stratum corneum une différence significative de pénétration cutanée a été observée entre les formules mousse de l'invention à 0.5% de tartrate de brimonidine composée du mélange des compositions A 3 et B 7 (dans un ratio 50/50 en poids) et le produit de référence gel contenant 0.5% de tartrate de brimonidine comme décrit dans la figure 3A. Une différence a également été observée dans les mêmes conditions dans l'épiderme comme décrit dans la figure 3A.

Dans le derme, les différences entre les mousses chimiques et les formes de gel de référence ont tendance à être réduites. Aucune différence significative n'a été observée entre les deux formes les deux concentrations testées comme illustré dans les figures 2B et 3B. Par ailleurs, aucune différence significative n'a été trouvée entre les concentrations testées au sein de la même forme (pas d'effet dose) suggérant que la variabilité des données empêche la signification statistique (test ANOVA test $F(3, 8) = 1{,}46$, $P = 0{,}296$).

La présente étude confirme l'obtention d'une mousse qui reste en surface de la peau et délivrer une quantité moindre afin d'éviter les effets indésirables (effet rebond) et d'obtenir une mousse adaptée au traitement de la rosacée.

## Revendications

**1.** Composition auto-moussante contenant de la brimonidine destinée à une application topique non rincée, comprenant:

- au moins une composition intermédiaire B comprenant un agent générateur de gaz et au moins un agent gélifiant et/ou agent de suspension choisi parmi les polymères d'acide acrylique, les polysaccharides, la famille des aluminium magnésium silicates, la famille des amidons modifiés et la famille des carraghénanes,
- au moins une composition intermédiaire A comprenant un agent activateur de l'agent générateur de gaz et au moins un agent gélifiant et/ou agent de suspension choisi parmi les polysaccharides, la famille des aluminium magnésium silicates, la famille des amidons modifiés, la famille des carraghénanes et le PVA, et
- de la brimonidine ou l'un de ses sels pharmaceutiquement acceptables contenue dans l'une au moins desdites compositions intermédiaires A et B.

**2.** Composition selon la revendication précédente, **caractérisée en ce que** la brimonidine est contenue dans la composition intermédiaire A.

**3.** Composition selon l'une quelconque des revendications précédentes, **caractérisée en ce qu'**elle ne comprend pas de tensioactifs moussants, et en particulier pas de tensioactifs choisis parmi les tensioactifs anioniques, les tensioactifs cationiques, les tensioactifs amphotères et les tensioactifs non ioniques de la famille des alkylpolyglucosides, et des glucamides.

**4.** Composition selon l'une quelconque des revendications précédentes, **caractérisée en ce que** l'agent générateur de gaz est choisi parmi le bicarbonate de sodium, le bicarbonate de potassium, le carbonate de sodium, le carbonate de potassium et leurs mélanges, et de préférence l'agent générateur de gaz est le bicarbonate de sodium.

**5.** Composition selon l'une quelconque des revendications précédentes, **caractérisée en ce que** l'agent générateur de gaz est présent dans la composition intermédiaire B en une quantité allant de 1% à 10%, préférentiellement de 2% à 8% en poids, par rapport au poids de la composition intermédiaire B.

**6.** Composition selon l'une quelconque des revendications précédentes, **caractérisée en ce que** l'agent activateur de l'agent générateur de gaz est choisi parmi un acide, un sel de polyacide partiellement salifié, une solution tampon d'acide faible et de sa base conjuguée, et les mélanges de ces composés.

**7.** Composition selon la revendication précédente, **caractérisée en ce que** l'agent activateur de l'agent générateur de gaz est choisi parmi l'acide citrique, l'acide tartrique, l'acide malique, l'acide lactique, l'acide phosphorique et l'acide pyrophosphorique, et les sels de ces acides, et plus préférentiellement ledit agent activateur est choisi parmi :

- un tampon acide tartrique/sel de tartrate ;
- un tampon acide citrique/citrate de sodium seul ;
- l'acide phosphorique, le phosphate de sodium, le disodium pyrophosphate seuls ou en mélange avec un tampon acide citrique/ citrate de sodium.

**8.** Composition selon la revendication précédente, **caractérisée en ce que** l'agent activateur de l'agent générateur de gaz est un tampon acide citrique/ citrate de sodium seul ou en mélange avec du phosphate sodique et/ou du disodium pyrophosphate.

**9.** Composition selon l'une quelconque des revendications précédentes, **caractérisée en ce que** la brominidine est sous forme de tartrate de brimonidine.

**10.** Composition sous forme de mousse, **caractérisée en ce qu'**elle résulte du mélange des compositions intermédiaires A et B telles que définies dans l'une quelconque des revendications précédentes.

**11.** Composition selon l'une quelconque des revendications précédentes, **caractérisée en ce qu'**elle contient en outre

un ou plusieurs actifs choisi parmi les agents émollients, les agents humectants, les agents anti-radicalaires, les agents anti-inflammatoires, les vitamines, les agents dépigmentants, les agents anti-acnéiques, les agents anti-séborrhiques, les agents anti-fongiques, les agents kératolytiques, les filtres solaires, les agents amincissants, les agents de coloration de la peau.

**12.** Médicament destiné à une application topique sur la peau, comprenant une composition telle que définie dans l'une quelconque des revendications 1 à 11.

**13.** Kit ou contenant unique à plusieurs compartiments comprenant de manière séparée au moins deux compositions intermédiaires:

- une composition intermédiaire A comprenant au moins un agent activateur de l'agent générateur de gaz, telle que définie dans l'une quelconque des revendications 1 à 11; et
- une composition intermédiaire B comprenant au moins un agent générateur de gaz telle que définie dans l'une quelconque des revendications 1 à 11 ;
- de la brimonidine ou l'un de ses sels pharmaceutiquement acceptables étant contenu dans l'une au moins desdites compositions intermédiaires A et B.

**14.** Kit ou contenant unique à plusieurs compartiments selon la revendication 13, **caractérisé en ce qu'**il est conçu pour mélanger les compositions intermédiaires A et B en un rapport en poids A/B allant de 0,5 à 2, préférentiellement de 0,5 à 1,5, plus préférentiellement de 0,9 à 1,1, et encore plus préférentiellement de 1.

**15.** Procédé de préparation d'une composition sous forme de mousse destinée à une application topique non rincée contenant de la brimonidine, dans lequel on mélange une composition intermédiaire A telle que définie dans l'une quelconque des revendications 1 à 11, avec une composition intermédiaire B telle que définie dans l'une quelconque des revendications 1 à 11, dans des proportions relatives en poids A/B allant de 0,5 à 2, préférentiellement de 0,5 à 1,5 et plus préférentiellement de 1.

## Patentansprüche

**1.** Selbstschäumende Zusammensetzung, die Brimonidin enthält, zur topischen Anwendung ohne Ausspülen, die Folgendes umfasst:

- mindestens eine Zwischenzusammensetzung B, die ein gaserzeugendes Mittel und mindestens ein Geliermittel und/oder Suspendiermittel, das aus Acrylsäurepolymeren, Polysacchariden, der Familie der Aluminiummagnesiumsilikate, der Familie der modifizierten Stärken und der Familie der Carrageenane ausgewählt ist, umfasst,
- mindestens eine Zwischenzusammensetzung A, die ein Aktivierungsmittel für das gaserzeugende Mittel und mindestens ein Geliermittel und/oder Suspendiermittel, das aus Polysacchariden, der Familie der Aluminiummagnesiumsilikate, der Familie der modifizierten Stärken, der Familie der Carrageenane und PVA ausgewählt ist, umfasst, und
- Brimonidin oder ein pharmazeutisch unbedenkliches Salz davon, das in mindestens einer der Zwischenzusammensetzungen A und B enthalten ist.

**2.** Zusammensetzung nach dem vorhergehenden Anspruch, **dadurch gekennzeichnet, dass** das Brimonidin in der Zwischenzusammensetzung A enthalten ist.

**3.** Zusammensetzung nach einem der vorhergehenden Ansprüche, **dadurch gekennzeichnet, dass** sie keine schäumenden Tenside und insbesondere keine Tenside, die aus anionischen Tensiden, kationischen Tensiden, amphoteren Tensiden und nichtionischen Tensiden aus der Familie der Alkylpolyglucoside und Glucamide ausgewählt sind, umfasst.

**4.** Zusammensetzung nach einem der vorhergehenden Ansprüche, **dadurch gekennzeichnet, dass** das gaserzeugende Mittel aus Natriumbicarbonat, Kaliumbicarbonat, Natriumcarbonat, Kaliumcarbonat und Mischungen davon ausgewählt ist und es sich vorzugsweise bei dem gaserzeugenden Mittel um Natriumbicarbonat handelt.

**5.** Zusammensetzung nach einem der vorhergehenden Ansprüche, **dadurch gekennzeichnet, dass** das gaserzeugende Mittel in der Zwischenzusammensetzung B in einer Menge von 1 bis 10 Gew.-%, vorzugsweise von 2 bis 8

Gew.-%, bezogen auf das Gewicht der Zwischenzusammensetzung B, vorliegt.

6. Zusammensetzung nach einem der vorhergehenden Ansprüche, **dadurch gekennzeichnet, dass** das Aktivierungsmittel für das gaserzeugende Mittel aus einer Säure, einem partiell versalzten Polysäuresalz, einer Pufferlösung einer schwachen Säure und ihrer konjugierten Base und Mischungen dieser Verbindungen ausgewählt ist.

7. Zusammensetzung nach dem vorhergehenden Anspruch, **dadurch gekennzeichnet, dass** das Aktivierungsmittel für das gaserzeugende Mittel aus Citronensäure, Weinsäure, Äpfelsäure, Milchsäure, Phosphorsäure und Pyrophosphorsäure und den Salzen dieser Säuren ausgewählt ist und weiter bevorzugt das Aktivierungsmittel aus

   - einem Weinsäure/Tartratsalz-Pulver;
   - einem Citronensäure/Natriumcitrat-Pulver allein;
   - Phosphorsäure, Natriumphosphat, Dinatriumpyrophosphat allein oder im Gemisch mit einem Citronensäure/Natriumcitrat-Puffer

   ausgewählt ist.

8. Zusammensetzung nach dem vorhergehenden Anspruch, **dadurch gekennzeichnet, dass** es sich bei dem Aktivierungsmittel für das gaserzeugende Mittel um einen Citronensäure/Natriumcitrat-Puffer allein oder im Gemisch mit Natriumphosphat und/oder Dinatriumpyrophosphat handelt.

9. Zusammensetzung nach einem der vorhergehenden Ansprüche, **dadurch gekennzeichnet, dass** das Brimonidin in Form von Brimonidintartrat vorliegt.

10. Zusammensetzung in Schaumform, **dadurch gekennzeichnet, dass** sie aus dem Mischen der Zwischenzusammensetzung A und B nach einem der vorhergehenden Ansprüche resultiert.

11. Zusammensetzung nach einem der vorhergehenden Ansprüche, **dadurch gekennzeichnet, dass** sie außerdem einen oder mehrere Wirkstoffe, die aus Emollientien, Feuchthaltemitteln, Radikalfängern, entzündungshemmenden Mitteln, Vitaminen, Depigmentierungsmitteln, Antiaknemitteln, antiseborrhoischen Mitteln, Fungiziden Keratolytika, Sonnenschutzmitteln, Schlankmachern und Hautfärbemitteln ausgewählt sind, enthält.

12. Medikament zur topischen Anwendung auf der Haut, umfassend eine Zusammensetzung gemäß einem der Ansprüche 1 bis 11.

13. Kit oder einzelner Behälter mit mehreren Fächern, das/der in getrennter Form mindestens zwei Zwischenzusammensetzungen umfasst:

   - eine Zwischenzusammensetzung A, die mindestens ein Aktivierungsmittel für das gaserzeugende Mittel umfasst, gemäß einem der Ansprüche 1 bis 11 und
   - eine Zwischenzusammensetzung B, die mindestens ein gaserzeugendes Mittel umfasst, gemäß einem der Ansprüche 1 bis 11,
   - Brimonidin oder ein pharmazeutisch unbedenkliches Salz davon, das in mindestens einer der Zwischenzusammensetzungen A und B enthalten ist.

14. Kit oder einzelner Behälter mit mehreren Fächern nach Anspruch 13, **dadurch gekennzeichnet, dass** es/er dafür ausgelegt ist, die Zwischenzusammensetzungen A und B in einem Gewichtsverhältnis A/B im Bereich von 0,5 bis 2, bevorzugt von 0,5 bis 1,5, weiter bevorzugt von 0,9 bis 1,1 und noch weiter bevorzugt von 1 zu mischen.

15. Verfahren zur Herstellung einer Zusammensetzung in Schaumform zur topischen Anwendung ohne Ausspülen, die Brimonidin enthält, bei dem man eine Zwischenzusammensetzung A gemäß einem der Ansprüche 1 bis 11 mit einer Zwischenzusammensetzung B gemäß einem der Ansprüche 1 bis 11 in relativen Gewichtsanteilen A/B im Bereich von 0,5 bis 2, weiter bevorzugt von 0,5 bis 1,5 und noch weiter bevorzugt von 1 mischt.

## Claims

1. A self-foaming composition containing brimonidine, intended for leave-on topical application, comprising:

- at least one intermediate composition B comprising a gas-generating agent and at least one gelling agent and/or suspending agent chosen from acrylic acid polymers, polysaccharides, the family of magnesium aluminum silicates, the family of modified starches and the family of carrageenans,
- at least one intermediate composition A comprising an agent for activating the gas-generating agent and at least one gelling agent and/or suspending agent chosen from polysaccharides, the family of magnesium aluminum silicates, the family of modified starches, the family of carrageenans and PVA, and
- brimonidine or one of its pharmaceutically acceptable salts contained in at least one of the said intermediate compositions A and B.

**2.** The composition as claimed in the preceding claim, **characterized in that** brimonidine is contained in the intermediate composition A.

**3.** The composition as claimed in either of the preceding claims, **characterized in that** it does not comprise any foaming surfactants, and in particular no surfactants chosen from anionic surfactants, cationic surfactants, amphoteric surfactants and nonionic surfactants of the family of alkylpolyglucosides and glucamides.

**4.** The composition as claimed in any one of the preceding claims, **characterized in that** the gas-generating agent is chosen from sodium bicarbonate, potassium bicarbonate, sodium carbonate and potassium carbonate, and mixtures thereof, and preferably the gas-generating agent is sodium bicarbonate.

**5.** The composition as claimed in any one of the preceding claims, **characterized in that** the gas-generating agent is present in the intermediate composition B in an amount ranging from 1% to 10% by weight and preferentially from 2% to 8% by weight, relative to the weight of the intermediate composition B.

**6.** The composition as claimed in any one of the preceding claims, **characterized in that** the agent for activating the gas-generating agent is chosen from an acid, a partially salified polyacid salt, a buffer solution of a weak acid and of its conjugate base, and mixtures of these compounds.

**7.** The composition as claimed in the preceding claim, **characterized in that** the agent for activating the gas-generating agent is chosen from citric acid, tartaric acid, malic acid, lactic acid, phosphoric acid and pyrophosphoric acid, and the salts of these acids, and more preferentially said activating agent is chosen from:

- a tartaric acid/tartrate salt buffer;
- a citric acid/sodium citrate buffer alone;
- phosphoric acid, sodium phosphate, disodium pyrophosphate, which are alone or as a mixture with a citric acid/sodium citrate buffer.

**8.** The composition as claimed in the preceding claim, **characterized in that** the agent for activating the gas-generating agent is a citric acid/sodium citrate buffer, alone or as a mixture with sodium phosphate and/or disodium pyrophosphate.

**9.** The composition as claimed in any one of the preceding claims, **characterized in that** brominidine is in the form of brimonidine tartrate.

**10.** A composition in foam form, **characterized in that** it results from the mixing of said intermediate compositions A and B as defined in any one of the preceding claims.

**11.** The composition as claimed in any one of the preceding claims, **characterized in that** it also contains one or more active agents chosen from emollients, humectants, free-radical scavengers, anti-inflammatory agents, vitamins, depigmenting agents, antiacne agents, antiseborrheic agents, antifungal agents, keratolytic agents, sunscreens, slimming agents and skin-coloring agents.

**12.** A medicament intended for topical application to the skin, comprising a composition as defined in any one of claims 1 to 11.

**13.** A kit or single multi-compartment container separately comprising at least two intermediate compositions:

- an intermediate composition A comprising at least one agent for activating the gas-generating agent, as defined

in any one of claims 1 to 11; and
- an intermediate composition B comprising at least one gas-generating agent as defined in any one of claims 1 to 11;
- brimonidine or a pharmaceutically acceptable salt thereof being contained in at least one of said intermediate compositions A and B.

**14.** The kit or single multi-compartment container as claimed in claim 13, **characterized in that** it is designed for mixing the intermediate compositions A and B in an A/B weight ratio ranging from 0.5 to 2, preferentially from 0.5 to 1.5, more preferentially from 0.9 to 1.1 and even more preferentially 1.

**15.** A process for preparing a composition in foam form intended for leave-on topical application, containing brimonidine, in which an intermediate composition A as defined in any one of claims 1 to 11 is mixed with an intermediate composition B as defined in any one of claims 1 to 11, in relative weight proportions A/B ranging from 0.5 to 2, preferentially from 0.5 to 1.5 and more preferentially 1.

FIG. 1

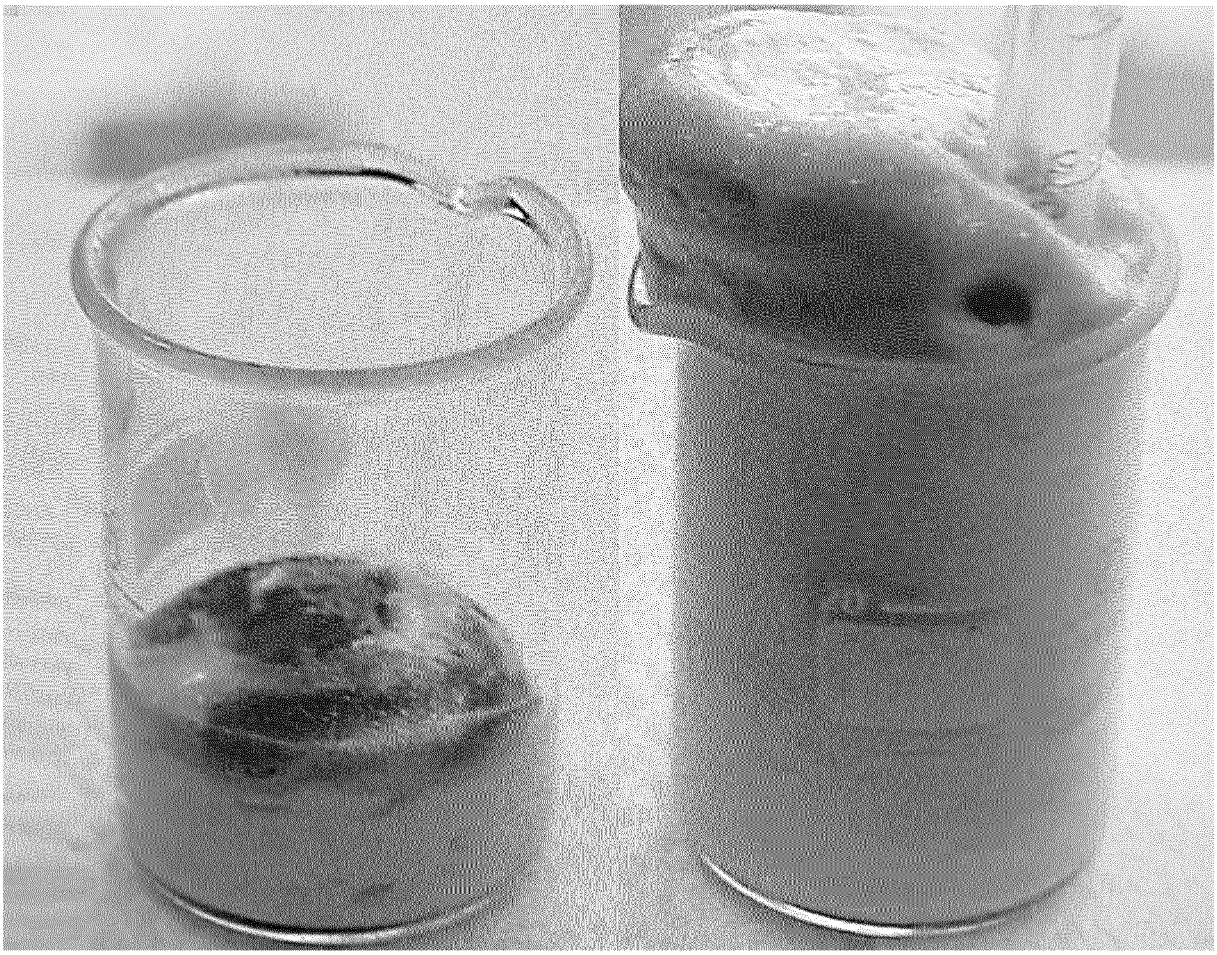

FIG 2A

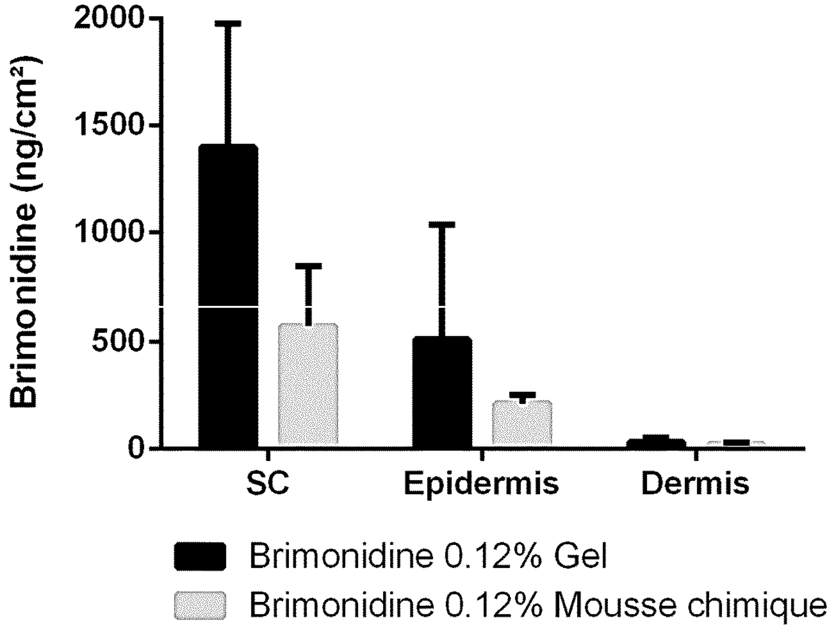
2000
1500
1000
500
0
Brimonidine (ng/cm²)
SC
Epidermis
Dermis
Brimonidine 0.12% Gel
Brimonidine 0.12% Mousse chimique

FIG. 2B

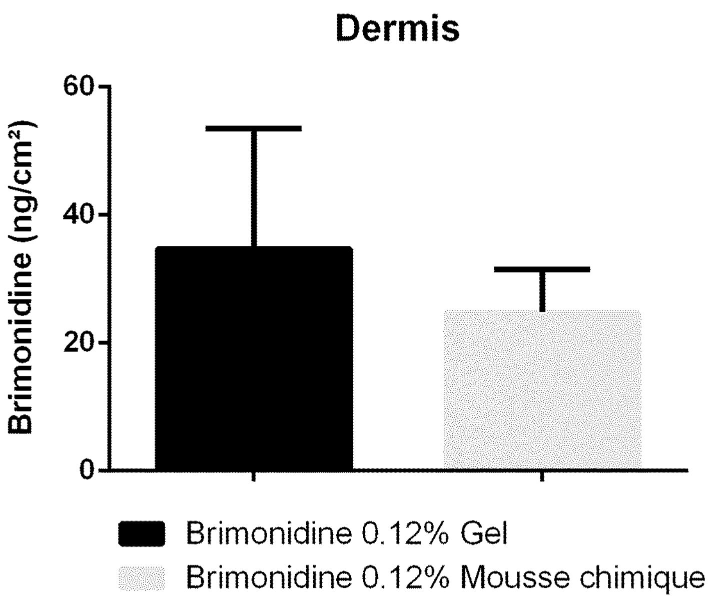
Dermis
60
40
20
0
Brimonidine (ng/cm²)
Brimonidine 0.12% Gel
Brimonidine 0.12% Mousse chimique

FIG. 3A

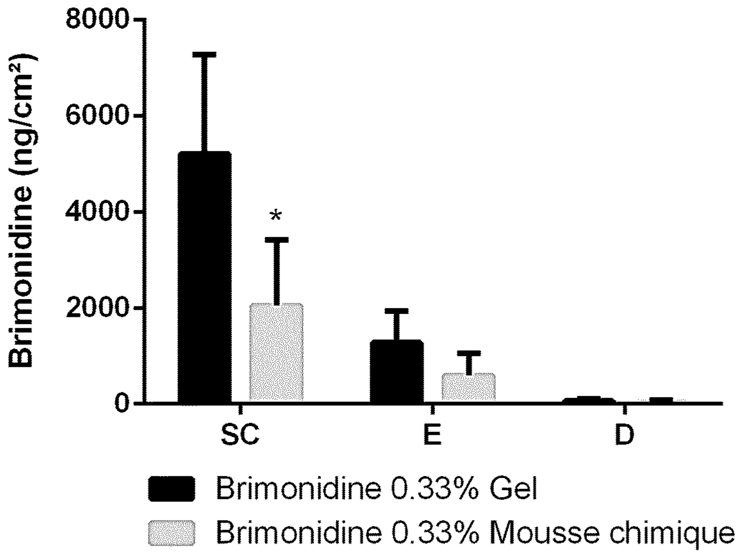
Brimonidine (ng/cm²)
8000
6000
4000
2000
0
*
SC
E
D
Brimonidine 0.33% Gel
Brimonidine 0.33% Mousse chimique

FIG. 3B

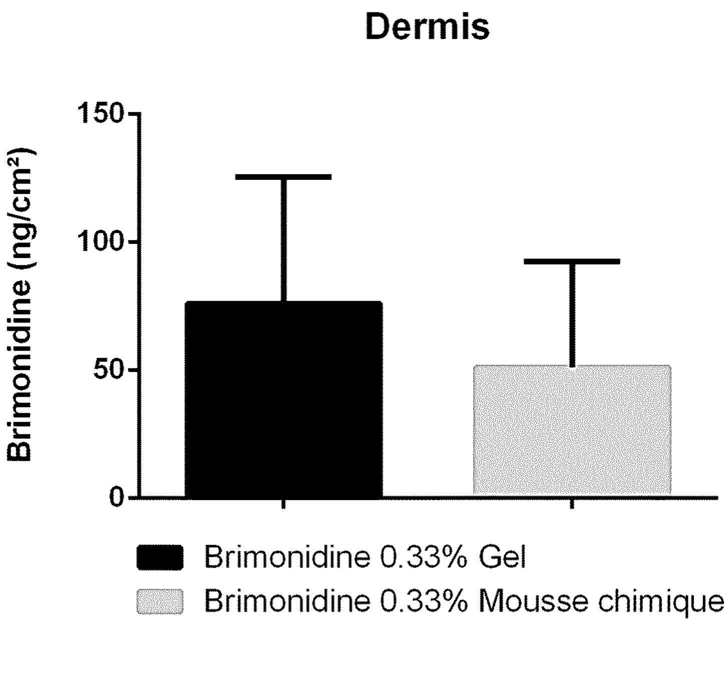
Dermis
Brimonidine (ng/cm²)
150
100
50
0
Brimonidine 0.33% Gel
Brimonidine 0.33% Mousse chimique